# EUROPEAN PATENT SPECIFICATION

(11) **EP 1 051 595 B1**
(45) Date of publication and mention of the grant of the patent: **09.07.2003**
(21) Application number: 99900202.5
(22) Date of filing: 13.01.1999
(51) Int. Cl.: G01B 11/10, G01N 33/36, G01N 21/89, D01H 13/26

(54) **METHOD AND DEVICE FOR DETERMINING THE THICKNESS OF A MOVING LINEAR TEXTILE FORMATION**
VERFAHREN UND VORRICHTUNG ZUM BESTIMMEN DER DICKE EINER BEWEGTEN LINEAREN TEXTILFORMATION
PROCEDE ET DISPOSITIF POUR LA DETERMINATION DE L'EPAISSEUR D'UNE FORMATION TEXTILE A MOUVEMENT LINEAIRE

(30) Priority: 14.01.1998 CZ 11398
(43) Date of publication of application: 15.11.2000
(73) Proprietor: Rieter CZ a.s., 562 15 Usti nad Orlici (CZ)
(72) Inventor: STUSAK, Miroslav;, 561 12 Brandys nad Orlici (CZ)
(74) Representative: Musil, Dobroslav, Dipl.-Ing.
(86) International application number: CZ9900002
(87) International publication number: WO99036746

(56) References cited:
- EP-A- 0 250 089
- EP-A- 0 271 728
- WO-A-95/13519
- FR-A- 2 749 932
- GB-A- 2 064 106
- US-A- 4 511 253
- US-A- 5 319 578
- PATENT ABSTRACTS OF JAPAN vol. 009, no. 265 (P-399), 23 October 1985 & JP 60 114704 A (FURUKAWA DENKI KOGYO KK), 21 June 1985

## Description

### Technical Field

The invention relates to a method of determining the thickness of a moving linear textile formation in which the linear textile formation moves in a radiation flux between a radiation source and a CCD sensor of radiation sensing the shape of the moving linear textile formation by evaluating the irradiance state of each element of the CCD sensor whereupon the real thickness of the linear textile formation is determined on the basis of the number of the shadowed elements.

The invention also relates to a device for carrying out the method comprising a radiation source emitting a radiation flux and a CCD sensor of radiation situated in the radiation flux, between which and the radiation source lies the path of the moving linear textile formation, the CCD sensor of radiation having related thereto an evaluation device containing an information output on the thickness of the measured linear textile formation and also having an auxiliary information output for controlling the radiation source.

### Background Art

Well-known devices used for monitoring a moving linear material such as yarn on a textile machine, comprise a light radiation source and receiver and are used for monitoring the yarn presence, as disclosed for instance in the CZ 281815.

Similar optical devices are used also for monitoring the thickness and/or homogeneity of yarn moving in a defined light field emitted by a well-known light radiation source such as a bulb, a. light emitting diode, a laser etc. Situated opposite the light radiation source is a light receiver such as a photoresist, a photodiode or a phototransistor or a linear set of light receivers as disclosed, for instance, in the US 4,511,253. The diameter of the yarn shadows a part of the light flux between the light source and the light receiver so that variations in the yarn diameter result in corresponding modifications in the output electrical parameters of the light receiver serving then for evaluating the variations in the yarn diameter. However, the well-known devices can determine only the variations in the yarn diameter, not the actual yarn diameter itself. Since they are used in textile plants, their function and precision are adversely influenced by the dustiness existing in such plants. In the operation of such devices, other negative causes such as the ageing of optical elements or the outer lighting and its variations cannot be eliminated. Another drawback consists in the indispensable calibration to the same sensitivity of the devices during their production, even in cases in which the production uses selected optical elements of analogical properties. As far as the uniformity is concerned, the optical methods can measure the uniformity in diameter represented by variations in the diameter of the moving linear formation. The mass uniformity, i.e., the homogeneity, of a moving linear formation cannot be measured by well-known optical methods, and capacity transducers are used for this purpose.

Another well-known method of measuring the profile uses a device comprising a radiation source and a CCD sensor of radiation between which the yarn to be measured moves in a measuring area. US 4,764,876 measures the twisting and entwining of textured yarn by detecting the thickness variations of the yarn from its thick to its thin sections and inversely. This device can determine only the twist number of the yarn but no other yarn parameters such as the yarn diameter. The device is not sensitive to hair or loops on the yarn circumference.

US 5,319,578 describes a yarn profile analyzer comprising a projection space between a light source and a light receiver made either as a linear matrix of CCD receivers or as a plane matrix of CCD receivers. The yarn is led through the projection space by special means providing for its constant speed and tension in said projection space. The analyzer comprises means for determining the level of the light received by the matrix element in order to ascertain whether the element has been blocked by the yarn, as well as means for determining the widths of bounded yarn sections and of the output signal of the elements of the CCD matrix. The width determining means comprise means for determining the pair of the mutually most widely apart matrix elements blocked (shadowed) by the yarn. The yarn diameter is determined by the number of the CCD matrix elements blocked by the yarn. For hairy yarns or for yarns presenting loops on their surface, it is possible to increase the threshold value of the element irradiation at which the element is evaluated as blocked by the yarn. The device is intended for laboratory qualitative evaluation of a yarn already produced prior to its use for the production of textile fabrics and requires a direct inspection by the operator charged with setting the threshold values of irradiation of the CCD elements, the value of the number of monitoring operations per unit of length, etc.

The device is not suitable for measuring qualitative parameters of yarn while being produced or processed on a textile machine in textile plants because it can eliminate the influence neither of the dustiness in such plants nor of the ageing of optical elements. To be applied on each operating unit of a textile machine, all measuring devices of this kind would need to be calibrated in order to give the same information on a given yarn diameter. Each time after a time interval, the calibration would have to be repeated with the ensuing high machine maintenance costs and high requirements imposed upon the qualification of the attending staff. Another drawback would consist in the necessity to set the machine out of operation during the calibration of the measuring devices with inevitable production losses resulting from it. Like the other methods, this one is unable to measure the homogeneity of the moving yarn or of another textile formation.

Another known solution, disclosed in JP-A-60114704, describes a method of measurement of the diameter of a piece of wire by means of an image sensor and a light source with parallel radiation where the width of the shadow is constant even though the distance between the wire to be measured and the image sensor is changed. The output of the image sensor is via an amplifier connected with a peak detector taking from the varying signal of the image sensor in each time sequence only the peak maximum radiation value which is, however, not related to a specific irradiated element of the sensor. The peak value from the peak detector is compared with a reference voltage in the fault amplifier and the difference is amplified so as to create a differential output. The light source is fed from the output proportional to the differential output by the feed unit.

### Disclosure of the Invention

The drawbacks of the background art have been eliminated by a method of determining the thickness of a moving linear formation according to claim 1, wherein the irradiance degree of at least one of the irradiated elements of the CCD sensor is evaluated and the intensity of radiation emitted from the radiation source is controlled in dependence on a comparison between the irradiance degree of a chosen single one of the irradiated elements of the CCD sensor and a preset value of the irradiance degree so as to ensure a constant irradiance degree of the irradiated elements of the CCD sensor during the operation. These drawbacks have been overcome as well by a corresponding device according to claim 5.

This arrangement substantially suppresses the influence of the dustiness and completely eliminates the influence of the ageing of the elements of the device on the quality of measuring of the thickness and/or homogeneity of the moving linear formation.

On a textile machine comprising a plurality of operating units such as a spinning machine, the device can be used on all the operating units. The parameter corresponding to the required threshold value of the radiation being received is defined by the design of the device. In operation, each device installed on each operating unit has the same parameters and consequently displays the same measured values which may be used for controlling the machine. The external calibration of the device at certain service intervals, currently used in the devices of the background art, becomes thus absolutely unnecessary.

In order to increase the precision with which the real thickness of the moving linear formation is determined, the irradiance degree of the elements of the CCD sensor is preferably monitored and evaluated at the edges of the shape (image) of the moving linear textile formation. As a rule, the dimensions of the elements vary in size from units to tens of urn, and in number from tens to hundreds of thousands.

It also can be advantageous to monitor and evaluate the irradiance degree of the elements of the CCD sensor inside the edges of the moving textile linear formation thus determining the thickness and homogeneity of the moving textile linear formation.

According to another preferred embodiment of the method according to the invention there is evaluated the irradiance degree of the elements of the CCD sensor thus determining the homogeneity of the monitored section of the moving linear textile formation.

In this connection, for instance the size of the core of the moving linear textile formation can be determined by using the evaluated degree of irradiation of the elements for determining the border of the moving linear textile formation, inside which the irradiation degree is evaluated. This can be advantageously used for instance for monitoring the features of a core yarn.

The principle of the device for carrying out the method according to the invention consists in that the evaluation device comprises means for evaluating the irradiance degree of at least one of the irradiated elements of the CCD sensor and means for comparing the found irradiance degree of a chosen single one of the irradiated elements with a preset value of the irradiance degree for controlling the intensity of the radiation of the source, and for ensuring a constant in time irradiance degree of the irradiated elements of the CCD sensor during the operation.

Due to this, the device is able to change the radiation amount emitted by the radiation source and eliminates thus in a simple and inexpensive way the drawbacks of the prior art.

The device can be used for determining both the thickness of the moving linear textile formation by evaluating the irradiation state of the elements of the CCD sensor and the homogeneity of the moving linear textile formation by evaluating the irradiance degree of the elements of the CCD sensor as well as for combining at will these two possibilities for obtaining more exact results of monitoring the thickness and/or the homogeneity of the moving linear textile formation. Their application depends in particular on the kind and properties of the monitored moving linear textile formation and on the technological needs and possibilities of the device on which the monitoring is carried out. The device is intended for mass application on textile machines, in particular on spinning and winding machines.

### Description of the Drawings

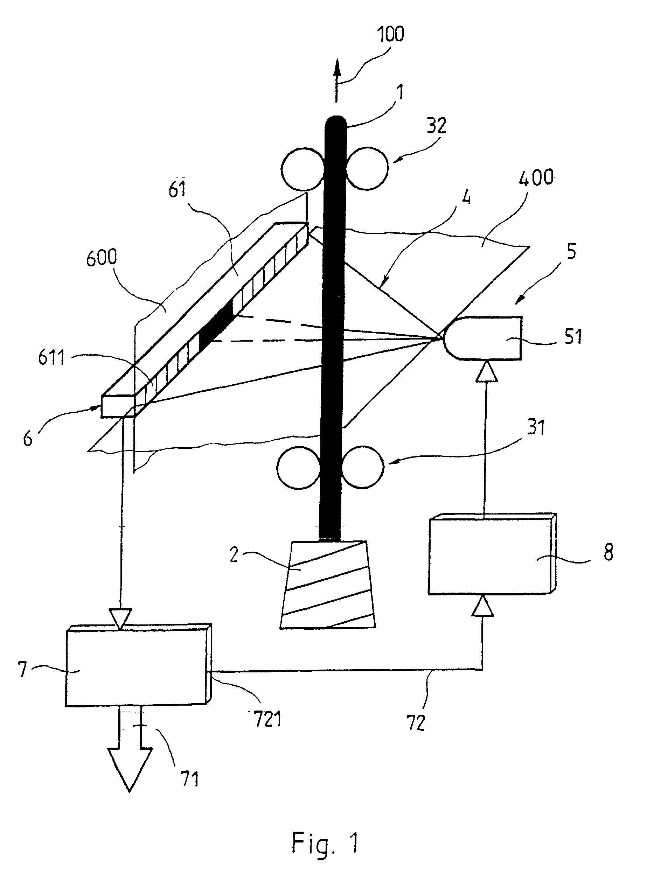

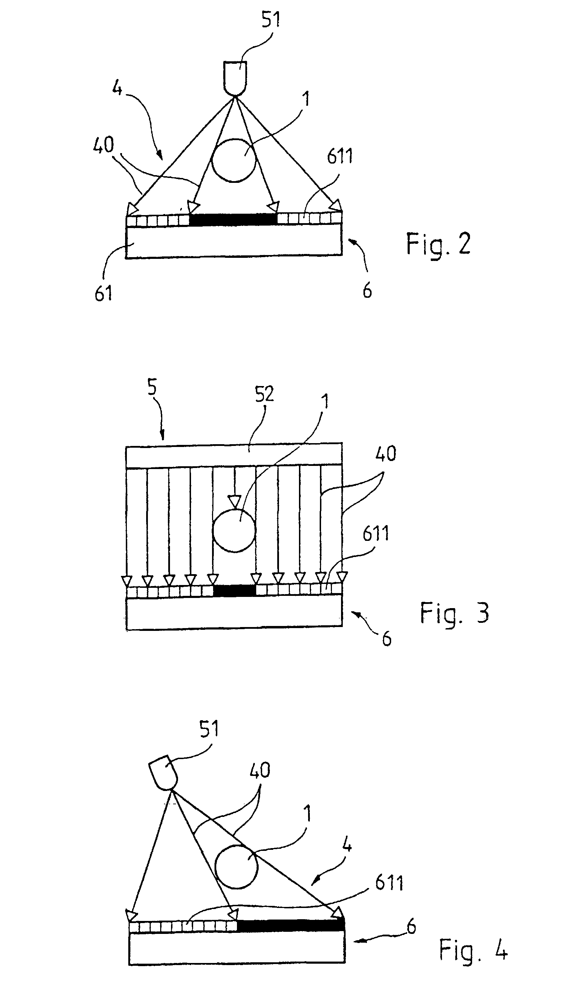

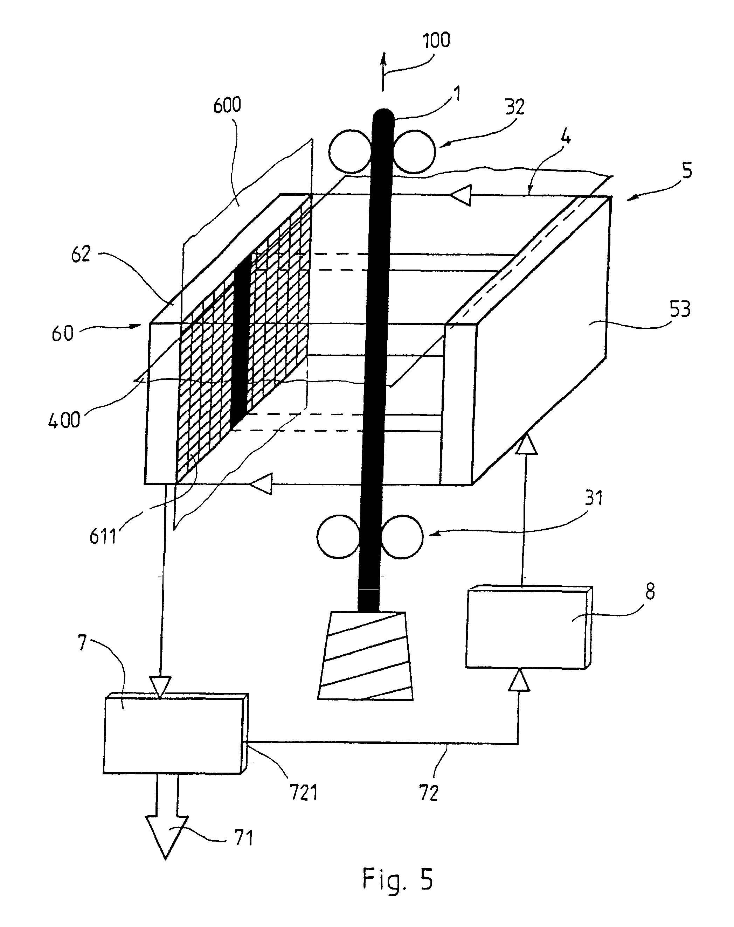

Embodiments of the device according to the invention are schematically shown on the annexed drawing in which Fig. 1 shows an axonometric view of the embodiment of the device equipped with a linear radiation receiver, Fig. 2 a section is the projection plane of the device according to Fig. 1, Fig. 3 a section in the projection line of an embodiment in which the plane passing through the axis of the linear formation and through the light source makes an acute angle with the projection plane, and Fig. 5 an axonometric view of the device equipped with a plane radiation receiver and a plane radiation source.

### Embodiments of the Invention

The method of determining the thickness of the moving linear textile formation such as yarn, thread, textile fibre, or textile fibre bundle will be described in more details relating to embodiments of the device for determining the thickness of yarn. The term "homogeneity" of the moving linear textile formation, as used throughout the text of this specification, is intended to mean its mass uniformity. The term "thickness" of the moving linear textile formation, as used throughout the text of this specification, is intended to mean the uniformity of its diameter, In the embodiment shown in Fig. 1, the moving linear textile formation consists of a yarn 1 delivered in a well-known manner from a well-known spinning device 2. The yarn 1 is delivered by a well-known delivery device acting in the represented embodiment also as a first stabilizing means 31 of the path of the yarn 1 consisting in the shown example of embodiment of a guide roller. Inserted into the path of the yarn 1 is in the shown embodiment a second stabilizing means 32 also consisting in the shown example of embodiment of a guide roller from which the yarn 1 is delivered in the direction of the arrow 100 to well-known not represented means for winding the yarn on a bobbin. As stabilizing means can be used also other well-known means or guiding the yarn, or the stabilizing means can be completely dispensed with.

Between the two stabilizing means 31, 32, the yarn 1 passes through a radiation flux 4 emitted by a radiation source 5 made in the embodiment shown in Fig. 1 as a one-point radiation source 51. Situated opposite the radiation source 5 is a radiation receiver v made as a linear CCD sensor 61 comprising a linear set of elements 611. Each of the set of the elements 611 of the CCD sensor 61 is connected with an evaluation device 7 of its irradiation state or its irradiance degree. Said evaluation device 7 can be made as an integral part of the CCD sensor 61 acting as the radiation receiver 6. The evaluation device 7 contains an output 71 furnishing information on the thickness and/or the homogeneity of the moving yarn 1. As dictated by specific needs of a given case, said output can be connected with devices intended to process information on the thickness and/or the homogeneity of the moving yarn 1 such as the control system of the machine or of the operating unit in question and/or with display devices and/or with recording devices and/or with adjusting means of the operating unit of the machine and/or with the control means of the operating unit serving to interrupt the moving yarn and/or to stop at least some of the functional sections of the operating unit of the machine, etc.

The evaluation device 7 is fitted with an auxiliary output 721 connected by an auxiliary conductor 72 with a radiation control device 8 controlling both the radiation intensity and the time course of radiation in a non-continuous radiation source connected with the radiation source 5. In some, not represented, embodiments, the radiation control device 8 is made as an integral part of the radiation source 5.

The one-point source 51 emits radiation and creates the radiation flux 4 incident on the elements 611 of the CCD sensor 6 acting as a radiation receiver. The path of the yarn 1 crosses the radiation flux 4. A monitoring plane 400 passes through the one-point radiation source 51 and through all radiationsensitive elements 611 of the radiation receiver 6.

The yarn 1 lying in the radiation flux 4 and moving along its longitudinal axis shadows some of the elements 611 of the CCD sensor 61 receiving the radiation. The evaluation device 7 evaluates the irradiation state (irradiated/non irradiated) of each of the elements 611 of the CCD sensor 61 and defines in this way the border of the image of the yarn 1. The number of shadowed elements 611 in combination with the knowledge of the geometrical situation existing between the one-point radiation source 51, the path of the yarn 1, the radiation receiver 6 in the monitoring plane 400, and the size of the elements 611 of the radiation receiver 6 serves as the basis for calculating the real thickness of the yarn 1 in the chosen length units such as µm. On the border of the image of the yarn 1 there will be evaluated the irradiance degree of the elements 611 situated at said border as a basis for determining the shadowed portion of the length of the element 611 in question and thus determining more exactly the actual thickness of the yarn 1.

At the same time, the irradiance degree of the shadowed elements 611 situated inside said border can be evaluated and compared with the irradiance degree of the irradiated elements 611 as a basis for determining the diameter uniformity and/or the homogeneity, of the yarn 1.

In technological processes requiring the monitoring of the moving linear textile formation such as a sliver of textile fibres, a core-yarn or a yarn only slightly twisted with loops or hair on its surface, the object of evaluation is the irradiance degree of the elements 611 of the CCD sensor 61 of the radiation receiver 6, permitting to determine the homogeneity of the monitored section of the moving linear textile formation after evaluating the degree of shadowing in the at least partly shadowed part of the set of the elements 611, of the CCD sensor.

At the same time, the evaluated irradiance degree of the radiation-sensitive elements 611 of the linear CCD sensor 61 permits to define the border of the moving linear formation and to evaluate the irradiation state of the radiationsensitive elements 611 and thus to determine, for instance, the dimension of the core of a moving core-yarn. The above methods of operation of the device can be combined at will according to the requirements of the technological process in question.

The device for determining the thickness and/or the homogeneity of the yarn shown in Fig. 1 can be modified for meeting specific requirements of some technological processes. The elements 611 remain in a line lying in the monitoring plane 400 and outside the path of the moving yarn 1 which is in the section of the monitoring plane 400 and the one-point radiation source 51 of Fig. 1, as shown in Fig. 2. The one-point radiation source 51 lies in the plane passing through the longitudinal axis of the yarn 1 and perpendicular to the line in which the elements 611 of the CCD sensor 61 of the radiation receiver. Fig. 3 shows another modification of the device of the Fig. 1 in the section of the monitoring plane 400. In this modification, the one-point radiation source 51 is replaced by a linear radiation source 52. The radiation flux 4 consists of parallel rays 40 so that this embodiment is suitable in particular for relatively thick yarn or other linear formation, because the shape (image) of the yarn 1 on the radiation receiver 6 consisting of a set of radiation-sensitive elements 611 is equal to the thickness of the linear formation situated in the radiation flux 4.

For technological reasons, it may be advantageous in some cases to situate the one-point radiation source 51 outside the plane perpendicular to the radiation receiver 6 and passing through the longitudinal axis of the linear formation as shown in Fig. 4.

In all the variants described above, both the evaluation of the irradiation state and the evaluation of the irradiance degree, of the radiation-sensitive elements 611 can be applied singly or in combination.

The evaluation device 7 comprises means for evaluating the irradiance degree of at least one of the irradiated elements 611 of the CCD sensor 61. The found irradiance degree, i.e., the amount of light received by a chose single one of the irradiated elements, is compared in the evaluation device 7 by one of the well-known methods with a preset threshold value of the received radiation. Depending on the results of this comparison, the evaluation device 7 sends at its auxiliary output 721 a control signal led by the auxiliary conductor 72 to the radiation control device 8 that uses the control signal to modify the radiation amount, i.e., the intensity or the time course of the radiation emitted by the radiation source. In this way, a constant irradiance degree of the irradiated elements 611 of the CCD sensor 61 is obtained.

Since, in most cases, the yarn 1 or another moving linear textile formation moves at a known speed and the evaluation of the irradiation state or of the irradiance degree of the radiation-sensitive elements takes a time interval, the monitoring of the thickness and/or of the homogeneity of the yarn by this method is a non-continuous one. In spite of this, it provides exact information on the actual thickness and/or homogeneity of the yarn 1 or of another linear textile formation in each of the subsequent monitoring planes. In a stabilized state, the distance between the monitoring planes 400 is constant but it can change, for instance decrease, upon the detection of a sudden change of the thickness and/or homogeneity of the yarn 1.

However, in some technological processes, it is required or more advantageous to monitor the moving linear textile formation continuously by monitoring the moving linear textile formation in a number of monitoring planes and projecting it into a projecting plane which crosses the monitoring planes which in their turn cross the path of motion of the moving linear textile formation. By evaluating the irradiation state and/or the irradiance degree of the radiationsensitive elements in each of the monitoring planes, the real thickness and/or homogeneity values of the moving linear textile formation in each of the monitoring planes are established and then assembled to the information on the actual shape and/or homogeneity of the moving linear textile formation in its monitored section. If the monitored sections of the moving linear textile formation follow or overlay each other, information on the actual shape and/or homogeneity of the whole moving linear textile formation is obtained.

An embodiment of a device for continuous monitoring of a moving linear textile formation will be described with reference to Fig. 5 in which the moving linear textile formation consists of the yarn 1, like in Fig. 1 moving in the direction of the arrow 100 and having its path stabilized by the first stabilizing means 31 and the second stabilizing means 32. The two stabilizing means 31, 32 can consist of any suitable yarn guide such as a guide roller etc. In some cases in which the path of the moving yarn 1 is sufficiently defined by the means of the operating unit of the machine, no stabilizing means are needed or they can consist of the components of the operating unit of the machine.

The yarn 1 is situated between the stabilizing means in the radiation flux 4 emitted by the radiation source 5 made in the represented embodiment as a plane radiation source 53. Situated opposite the radiation source 5 is the radiation receiver 6 made as a matrix CCD sensor 62 comprising in the shown embodiment a number of line sets of the elements 611 arranged on top of each other, with the elements 611 arranged side by side. The elements 611 are arranged in a projection plane 600 lying outside the path of the moving yarn 1 and in the shown embodiment parallel with the path of the yarn 1. The radiation flux 4 can be subdivided into a number of monitoring planes 400 intersecting with the projecting plane 600 in lines in which the sets of the elements 611 are situated. Like in the embodiment shown in Fig. 1, each of the elements 611 of the radiation receiver 6 is coupled with the evaluation device 7 evaluating its irradiation state and/or its irradiance degree, and the evaluation device 7 can be made as an integral part of the radiation receiver 6. The evaluation device 7 is equipped with the output 71 of information on the thickness and/or homogeneity of the monitored section of the yarn 1. This output 71 can be connected with a device intended to process the information on the thickness and/or homogeneity of the moving yarn 1, identical with those of the embodiment shown in Fig. 1.

The evaluation device 7 is also in the embodiment shown in Fig. 5 fitted with the auxiliary output 721 connected by means of the auxiliary conductor 72 with the radiation control device. The radiation control device 8 is connected with the radiation source 5 or is made as an Integral part thereof.

The plane radiation source 53 produces the radiation flux 4 whose rays are parallel and incident on the radiation-sensitive elements 611 of the matrix CCD sensor 62 constituting the radiation receiver 6, and create so a number of parallel monitoring planes 400 crossing the path of the yarn 1 moving in the direction of the arrow 100. In each of the monitoring planes 400, the yarn 1 shadows some of the elements 611 of a given line of elements 611 of the CCD sensor 62, thus producing the image of the monitored section of the yarn 1 on the projection plane 600.

The evaluation of the irradiation state or of the irradiance degree of the radiation-sensitive elements 611 of the matrix CCD sensor 62 of the radiation receiver 6 is carried out in the evaluation device 7 in the same manner as in the example of embodiment shown in Fig. 1.

The information on the thickness and/or homogeneity of the monitored section of the yarn 1 is obtained by combining the information on the actual thickness and/or homogeneity of the yarn in each of the monitoring planes 400 of the monitored section of the yarn 1, or in each line of the elements of the matrix CCD sensor. In another embodiment, the set of information items on the actual thickness and/or homogeneity of the yarn in each of the monitoring planes 400 of the monitored section of the yarn 1 is used as a basis for calculating the average values of the thickness and/or homogeneity of the monitored section of the yarn 1 which in repeated monitoring serve for the non-continuous monitoring of the thickness and/or homogeneity of the moving yarn 1 or of another linear formation.

The invention in not limited to the described specific embodiments. As required bu the design of the device according ti the invention or by the design of the machine on which the device according to the invention is to be applied or by the requirements of the technological process, the described examples of embodiment of the device for determining the thickness and/or the homogeneity of the moving linear textile formation can be mutually combined. Also, the elements of the method and of the device can be substituted by equivalent elements.

## Claims

1. A method of determining the thickness of a moving linear textile formation in which the linear textile formation moves in a radiation flux (4) between a radiation source (5) and a CCD sensor (61) of radiation sensing the shape of the moving linear textile formation by evaluating the irradiance state of the elements (611) of the CCD sensor (61) whereupon the real thickness of the linear textile formation is determined on the basis of the number of the shadowed elements, **characterized by** that the irradiance degree of at least one of the irradiated elements (611) of the CCD sensor (61) is evaluated and the radiation intensity emitted by the radiation source (5) is controlled in dependence on a comparison between the irradiance degree of a chosen single one of the irradiated elements (611) of the CCD sensor (61) and a preset value of the irradiance degree so as to ensure a constant in time irradiance degree of the irradiated elements (661) of the CCD sensor (61) during the operation.

2. A method as claimed in Claim 1, **characterized by** monitoring and evaluating the irradiance degree of the elements (611) of the CCD sensor (61) at edges of the image of the moving linear textile formation in order to increase the precision with which the real thickness of the moving linear textile formation is determined.

3. A method as claimed in Claim 1 or 2, **characterized by** monitoring and evaluating the irradiance degree of the elements (611) of the CCD sensor (61) inside the edges of the image of the moving linear textile formation in order to determine the thickness and homogeneity of the moving linear textile formation.

4. A method as claimed in Claim 1, **characterized by** evaluating the irradiance degree of all the elements (611) of the CCD sensor as a basis for determining the homogeneity of the monitored section of the-moving linear textile formation.

5. A device for carrying out the method according to one of the claims 1 to 4, comprising a radiation source (5) emitting a radiation flux (4) and a CCD radiation sensor (61) situated in the radiation flux (4), the device being arranged so that the path of the moving linear textile formation lies between said CCD radiation sensor (61) and the radiation source (5), the CCD radiation sensor (61) having related thereto an evaluation device (7) containing an output (71) of information on the thickness of the measured linear textile formation and also having an auxiliary output (721) of a signal for controlling the radiation source (5) **characterized by** that the evaluation device (7) comprises means for evaluating the irradiance degree of at least one of the irradiated elements (611) of the CCD sensor (61) and means for comparing the found irradiance degree of a chosen single one of the irradiated elements (611) with a preset value of irradiance degree for controlling the intensity of the radiation of the source (5) and for ensuring a constant in time irradiance degree of the irradiated elements (611) of the CCD sensor (61) during the operation.

## Patentansprüche

1. Ermittlungsverfahren der Dicke des bewegten linearen Textilgebildes, bei der sich ein lineares Textilgebilde im Strahlungsfluss (4) zwischen der Strahlungsquelle (5) und dem CCD-Strahlungssensor (61) bewegt, der die Form des bewegten linearen Textilgebildes durch Zustandsauswertung von Bestrahlung einzelner Elemente (611) des CCD-Sensors (61) abtastet, wonach aus der Anzahl von beschatteten Elementen (611) die Istdicke des linearen Textilgebildes ermittelt wird, **dadurch gekennzeichnet, dass** das Bestrahlungsniveau zumindest eines der bestrahlten Elemente (611) des CCD-Sensors (61) ausgewertet wird, und die von der Strahlungsquelle (5) emittierte Strahlungsstärke abhängig von Vergleich des Bestrahlungsniveaus eines gewählten der bestrahlten Elemente (611) des CCD-Sensors (61) und des voreingestellten Werts des Bestrahlungsniveaus gesteuert wird, wodurch während des Betriebs Bestrahlungsniveaus der bestrahlten Elemente (661) des CCD-Sensors (61) konstant gehalten wird.

2. Ermittlungsverfahren nach dem Anspruch 1, **dadurch gekennzeichnet, dass** die Verfolgung und die Auswertung vom Bestrahlungsniveau der Elemente (611) des CCD-Sensors (61) an den Bildgrenzen des bewegten linearen Textilgebildes, wodurch die Ermittlung von Istdicke des bewegten linearen Textilgebildes präzisiert wird.

3. Ermittlungsverfahren nach dem Anspruch 1 oder 2, **dadurch gekennzeichnet, dass** die Verfolgung und die Auswertung vom Bestrahlungsniveau der Elemente (611) des CCD-Sensors (61) innerhalb von Bildgrenzen des bewegten linearen Textilgebildes, wodurch die Dicke und die Homogenität des bewegten linearen Textilgebildes ermittelt werden.

4. Ermittlungsverfahren nach dem Anspruch 1, **dadurch gekennzeichnet, dass** die Auswertung vom Bestrahlungsniveau aller Elemente (611) des CCD-Sensors (61), woraus die Homogenität des abgetasteten Bereichs des bewegten linearen Textilgebildes ermittelt wird.

5. Vorrichtung zur Umsetzung des Verfahrens nach einem der Ansprüche 1 bis 4, bestehend aus der Strahlungsquelle (5), die den Strahlungsfluss (4) emittiert und dem CCD-Sensor (61) angeordnet im Strahlungsfluss (4), die Vorrichtung ist so gestaltet, dass der Weg des bewegten linearen Textilgebildes zwischen dem erwähnten CCD-Sensor (61) und der Strahlungsquelle (5) liegt, wobei dem CCD-Sensor (61) ein Auswertegerät (7) zugeordnet ist, das den Ausgang (71) von Daten zur Dicke des zu messenden linearen Textilgebildes beinhaltet und gleichzeitig über einen Hilfsausgang (721) des Signals für Ansteuerung der Strahlungsquelle (5) verfügt, **dadurch gekennzeichnet, dass** das Auswertegerät (7) Mittel für Auswertung von Bestrahlungsniveau zumindest eines der bestrahlten Elemente (611) des CCD-Sensors (61) und Mittel für Vergleich des ermittelten Bestrahlungsniveaus eines gewählten der bestrahlten Elemente (611) mit einem voreingestellten Wert des Bestrahlungsniveaus beinhaltet, um die Strahlungsstärke der Quelle (5) zu steuern und ein in der Zeit gleichbleibendes Bestrahlungsniveau der bestrahlten Elemente (611) des CCD-Sensors während des Betriebs zu halten.

## Revendications

1. Façon de détection de l'épaisseur de la figure textile linéaire en mouvement consistant dans le fait que la figure textile linéaire se déplace dans le flux (4) de rayonnement entre la source (5) de lumière et le capteur CCD (61) de rayonnement qui capte l'ombre de la figure textile linéaire en mouvement à l'aide de l'évaluation de l'état d'éclairage de différents éléments (611) du capteur CCD (61) après quoi l'épaisseur totale de la figure textile linéaire est déterminée à partir du nombre d'éléments ombrés (611) **caractérisée en ce que** le degré d'éclairage d'au moins un des éléments éclairés (611) du capteur CCD (61) est évalué et l'intensité du rayonnement émis par la source (5) de lumière est fonction de la comparaison du degré de l'éclairage d'un des éléments choisis (611) du capteur CCD et de la valeur prédéterminée de l'éclairage ce qui maintient le degré constant de l'éclairage des éléments (811) du capteur CCD (61) au cours de l'exploitaion.

2. Façon d'après la revendication 1 **caractérisée par** le suivi et l'évaluation du degré de l'éclairage des éléments (611) du capteur CCD (61) sur les limites de l'image en mouvement de la figure textile linéaire ce qui contribuera à la précision de la détermination de l'épaisseur réelle de la figure textile linéaire en mouvement.

3. Façon d'après les revendications 1 ou 2 **caractérisée par** le suivi et l'évaluation du degré de l'éclairage des éléments (611) du capetur CCD (61) à l'intérieur des limites de l'image de la figure textile linéaire en mouvement ce qui déterminera l'épaisseur et l'homogénéité de la figure textile linéaire en mouvement.

4. Façon d'après la revendication 1 **caractérisée par** l'évaluation du degré de l'éclairage de tous les éléments (611) du capteur CCD (61) à partir duquel l'homogénéité de la partie captée de la figure textile linéaire en mouvement est déterminée.

5. Dispositif pour la réalisation de la façon d'après une des revendications 1 à 4 contenant la source de lumière (5) organisée en flux (4) de rayonnement le dispositif étant disposé en sorte que le trajet de la figure textile linéaire en mouvement est située entre le capteur CCD (61) du rayonnement et la source (5) de lumière au capteur CCD (61) étant attribuée l'évaluation du rayonnement du dispositif (7) contenant la sortie (71) des informations sur l'épaisseur de la figure textile linéaire mesurée et contenant en même temps la sortie auxiliaire (721) du signal pour régler la source (5) de rayonnement **caractérisé en ce que** le dispositif d'évaluation (7) contient les moyens pour l'évaluation du degré d'éclairage d'au moins un des éléments éclairés (611) du capteur CCD (51) et les moyens pour la comparaison du degré constaté de l'éclairage d'un des éléments éclairés choisis (611) avec la valeur prédeterminée du degré de l'éclairage pour régler l'intensité du rayonnement de la source (5) et maintenir dans le temps le degré constant de l'éclairage des éléments éclairés (611) du capteur au cours de l'exploitation.
